# EUROPEAN PATENT SPECIFICATION

(11) **EP 2 027 159 B1**
(45) Date of publication and mention of the grant of the patent: **13.12.2017**
(21) Application number: 07795984.9
(22) Date of filing: 12.06.2007
(51) Int. Cl.: C08B 37/00, D21C 3/20, C08H 7/00, C08H 8/00, C13K 1/02, C12P 7/10, D21C 3/04

(54) **A PROCESS FOR THE STEPWISE TREATMENT OF LIGNOCELLULOSIC MATERIAL TO PRODUCE REACTIVE CHEMICAL FEEDSTOCKS**
VERFAHREN ZUR SCHRITTWEISEN BEHANDLUNG VON LIGNOCELLULOSEMATERIAL ZUR PRODUKTION REAKTIVER CHEMIEROHSTOFFE
PROCÉDÉ DE TRAITEMENT ÉTAPE PAR ÉTAPE D'UN MATÉRIAU LIGNOCELLULOSIQUE POUR PRODUIRE DES COMPOSÉS CHIMIQUES DE BASE RÉACTIFS

(30) Priority: 12.06.2006 US 812244 P; 12.06.2006 US 812245 P; 12.06.2006 US 812246 P; 05.07.2006 US 818342 P; 22.12.2006 US 876470 P; 27.04.2007 US 740923
(43) Date of publication of application: 25.02.2009
(73) Proprietor: American Process, Inc., Atlanta GA 30308 (US)
(72) Inventor: RETSINA, Dr. Theodora, Atlanta, Georgia 30308 (US); PYLKKANEN, Mr. Vesa, Atlanta, Georgia 30329 (US); RYHAM, Mr. Rolf, Suwanee, Georgia 30024 (US)
(74) Representative: Wilkins, Christopher George
(86) International application number: PCT/US2007/013715
(87) International publication number: WO 2007/146245

(56) References cited:
- EP-A1- 0 008 783
- US-A- 2 060 068
- US-A- 3 549 617
- US-A- 5 338 405
- US-A- 5 705 369
- US-A1- 2002 148 574
- US-B1- 6 258 175

## Description

### FIELD OF THE INVENTION

This invention relates, in general, to the fractionation of lignocellulosic material into lignin, cellulose and fermentable hemicelluloses, and more particularly to the production of reactive lignin and reactive cellulose in a semi-continuous or batch process. The reactive materials can be used as feedstock for a variety of chemical syntheses including alcohols, organic acids, polymers and other bioproducts.

### BACKGROUND OF THE INVENTION

Fractionation technologies of lignocellulosic material into its main subcomponents of cellulose, lignin and hemicelluloses have existed both in commercial practice and at the research level. The most prevalent of these are commercial sulfite pulping and the U.S. National Renewable Energy Laboratory, NREL, clean fractionation technology research.

Commercial sulfite pulping has been practiced since 1874. The focus of sulfite pulping is the preservation of cellulose which is produced in a crystalline non-reactive form. In an effort to do that, industrial variants of sulfite pulping use a single step process which takes 6 - 10 hours. In this prolonged processing the hemicelluloses and lignin are dissolved, some hemicelluloses are hydrolyzed into sugars, and then some sugars are converted to organic acids resulting in an overall a low yield of fermentable sugars and converting all lignin to lignosulfonates.

Sulfite pulping produces spent cooking liquor termed sulfite liquor. Fermentation of sulfite liquor to hemicellulosic ethanol has been practiced primarily to reduce the environmental impact of the discharges from sulfite mills since 1909. Published design data from one of the two known remaining sulfite mills that produces ethanol, shows ethanol yields not to exceed 33% of original hemicelluloses. Ethanol yield is low due to the incomplete hydrolysis of the hemicelluloses to fermentable sugars and further compounded by sulfite pulping side products, such as furfural, methanol, acetic acid and others, inhibiting fermentation to ethanol.

Because of poor ethanol yield, lower cost of synthetic ethanol production from oil feed stock, and the production of ethanol from corn today, only two sulfite mills are known to have continued the practice of hemicellulosic ethanol production to date.

In the 20^{th} century, Kraft pulping eclipsed sulfite pulping as the dominant chemical pulping method. Kraft pulping however does not fractionate lignocellulosic material into its primary components in a reactive form. Instead, hemicelluloses are in solution with soluble inorganic cooking chemicals and non-reactive lignin, (condensed lignin with no active site available for chemical bonding), and the lignin cannot readily be separated.

Other processes using solvent cooking chemicals have been tried as an alternative to Kraft or sulfite pulping. The original solvent process is described in U.S. Patent No. 1,856,567 to Kleinert et al. Although three demonstration size facilities for ethanol-water (ALCELL), alkaline sulfite with anthraquinone and methanol (ASAM), and ethanol-water-sodium hydroxide (Organocell) were operated briefly in the 1990's, today there are no full scale solvent pulping mills. Of these technologies only ALCELL produced native reactive lignin by the use of pure aqueous organic solvents in elevated thermodynamic conditions. None of these technologies produced reactive cellulose or hydrolyzed hemicelluloses.

Groombridge et al. in U.S. Patent No. 2,060,068 shows that an aqueous solvent with sulfur dioxide is a potent delignifying system to produce cellulose from lignocellulosic material. This process produces non-reactive cellulose for papermaking and the hemicelluloses and lignin are not fractionated.

Furthermore, U.S. Patent No. 5,879,463 to Proenca reveals that simultaneous delignification and rapid hydrolysis of the entire cellulosic material, both the cellulose and the hemicelluloses, is possible in the presence of an organic solvent and a dilute inorganic acid; however this process does not preserve the cellulose.

Finally, in U.S. Patent No. 5,730,837 to Black et al. claims fractionation of lignocellulosic material into lignin, cellulose and dissolved sugars using ketone, alcohol, water and mineral acid. This is more readily known as the NREL clean fractionation technology. The lignin so produced is not all in a reactive form because of the use of strong acid that condenses some of the lignin.

In most wood based biorefinery processes a major part of total consumed energy is used to concentrate the extracted sugars and/or organic components to a concentration that is useful for downstream processing. In the Kraft process this is done with multiple effect evaporators which use steam. In corn ethanol processes the distillation of ethanol and evaporation of water from the distillers grain is done in a two step process using steam. In US Patent 6,217,711 to Ryham et al, the stripping of methanol and the concentration of black liquor was achieved in an integrated process in which the liquor was first concentrated and the methanol was then stripped from it. In the present invention an integrated stripper and evaporator are designed to first remove aliphatic alcohol from a stream and then to concentrate the stream.

Therefore in the prior art of processing lignocellulosic material:
a) The sulfite processes to date preserves cellulose in a non-reactive form, degrades some hemicelluloses into non-reactive byproducts and sulfonates all lignin.
b) The Kraft process does not fractionate lignin, cellulose and hemicelluloses
c) Organic solvent pulping methods produced a non-reactive cellulose and did not hydrolyze hemicelluloses. Furthermore they used pressures and temperatures significantly higher than conventional pulping methods.
d) Treatment of lignocellulosic material with dilute inorganic acid in organic solvent hydrolyzes both cellulose and hemicelluloses and therefore does not preserve cellulose
e) Treatment of lignocellulosic material with ketone, alcohol, water and mineral acid does not produce all lignin in a reactive form.

The present inventors have now developed a process for the treatment of lignocellulosic material which first fractionates the material and then converts each fraction into a reactive chemical feedstock. This is achieved through cooking lignocellulosic material with sulfur dioxide in a solution of ethanol and water in a one or multiple stage process where treatment of each fraction is continued after intermediary separation of the fractions and such treatment conditions are modified to achieve desired fraction final properties. This can be done in a batch or semi continuous process.

Surprisingly, such a process can isolate at least four reactive components within one multiple stage integrated process treatment and provide four industrial feedstock chemicals in large scale.

These are:
1) cellulose which can be diverted to paper making or be further treated to produce reactive esterified cellulose suitable for industrial production of synthetic fibers and polymers, or for high yield ethanol production. In the esterification, cellulose is converted into an amorphous form where the free hydroxyl groups are exposed to further reaction.
2) fermentable hemicelluloses and fermentable sugars suitable for high yield ethanol production
3) reactive native lignin, i.e., lignin dissolved by alcoholysis and is in near original polymer length and its reactive sites are preserved in the solution
4) reactive lignosulfonates, i.e., lignin that has been partially sulfonated but retains near original polymer length and can further react with electrolytes.

Molecular sieves are the common method of breaking the water-ethanol azeotrope in corn ethanol plants. Molecular sieves consume at least 150 kilojoules of energy per liter of ethanol to remove the last 4.5% of water, which is more energy than traditional distillation of water. Use of lime for dehydrating ethanol is common practice in laboratories but an industrial scale application is cost prohibitive because of the cost of regenerating lime from its resultant hydrated state to the required anhydrous state, and the associated loss of ethanol captured in the hydrated lime. Coincidentally, it was realized in the present invention that the hydrated lime byproduct can be reintroduced to the process in the lignin precipitation step.

Therefore, the lime is beneficially used in the process and the ethanol captured in the hydrated lime is recovered to the process.

### BRIEF SUMMARY OF THE INVENTION

One aspect of the invention is a process for fractionating lignocellulosic material in to chemically reactive components through a staged treatment of the lignocellulosic material with a solution of aliphatic alcohol, water and 10-20% wt sulfur dioxide with intermediate separation of cellulose and hemicelluloses-lignin fractions which are then each further treated, wherein said process further comprises a step of lignin separation and a step wherein the hemicelluloses are converted into fermentable sugars.

Another aspect is a process wherein said solution of aliphatic alcohol, water and sulfur dioxide contains 40% to 60% water.

Another aspect is a process wherein a different concentration of said solution of aliphatic alcohol, water and sulfur dioxide is used at a first stage of treatment of said lignocellulosic material than is used in one or more subsequent stages of treatment with intermediate removal and preservation of cellulose.

Another aspect is a process wherein a sulfur dioxide solution of 10-20% wt is used at a first stage of treatment and a sulfur dioxide, sulfurous acid or sulfuric acid solution of 0.5% to 20% is used in one or more subsequent stages of treatment with intermediate removal and preservation of cellulose.

Another aspect is a process wherein said process is followed by steam stripping and/or evaporation of hydrolyzate to remove and recover sulfur dioxide and aliphatic alcohol and to remove fermentation inhibitors.

Another aspect is a process wherein cellulose is further treated with an aqueous solution of aliphatic alcohol in the presence of acid to esterify and render it a reactive chemical feedstock.

Another aspect is a process wherein the esterified cellulose is fermented to aliphatic alcohol.

Another aspect is a process wherein said acid is sulfur dioxide, sulfurous acid or sulfuric acid solution of 0.5% to 20%.

A process wherein conditions are 95% ethanol and 5% sulfuric acid at 120 °C is disclosed herein.

Another aspect is a process wherein said process is carried out at temperatures between 65 °C and 200 °C.

Another aspect is a process wherein said process is carried out for a period of time between 15 minutes and 720 minutes.

A process is disclosed herein wherein conditions are an initial treatment using 47% ethanol, 47% water and 6% sulfur dioxide at 140°C for 2 hours, and following the intermediate removal and preservation of the cellulose, a further treatment of the hemicelluloses-lignin fraction using 2% ethanol, 95% water and 3% sulfur dioxide at 140 °C for 1 hour.

A process is disclosed herein for producing fermentable sugars from the hemicelluloses of a lignocellulosic material through a staged treatment of the lignocellulosic material with a solution of aliphatic alcohol, water and sulfur dioxide with intermediate removal of hydrolyzate and cellulose.

Another aspect is a process wherein a different concentration of said solution of aliphatic alcohol, water and sulfur dioxide is used at a first stage of treatment of said lignocellulosic material than is used in one or more subsequent stages of treatment with intermediate removal of hydrolyzate and cellulose.

Another aspect is a process wherein aliphatic alcohol is produced from fermenting and distilling hydrolyzed fermentable sugars produced in said process and is then reused in said process.

Another aspect is a process wherein lignin is sulfonated and rendered soluble in aqueous solutions.

Another aspect is a process wherein the concentration of sulfur dioxide and aliphatic alcohol in the solution and the time of cook is varied to control the yield of hemicelluloses vs. celluloses and vs. fermentable sugars.

Another aspect is a process wherein excess sulfur dioxide is released from said further treatment of each fraction and used for make-up for cooking chemicals.

Another aspect is a process for fractionating lignocellulosic material in to chemically reactive components through a staged treatment of the lignocellulosic material with a solution of aliphatic alcohol, water and 10-20% wt sulfur dioxide with intermediate separation of cellulose and hemicelluloses-lignin fractions which are then each further treated, wherein said process further comprises a step of lignin separation and a step wherein the hemicelluloses are converted into fermentable sugars, comprising the steps of:
Cooking under acidic conditions to produce hydrolyzed hemicelluloses, cellulose, reactive lignin and sulfonated lignin;
Washing to separate lignin and hemicelluloses from cellulose in several stages to recover over 95% of the aliphatic alcohol mixed with the cellulose;
Diverting the cellulose to papermaking or treatment of cellulose with an aqueous solution of aliphatic alcohol in the presence of acid to esterify the cellulose, rendering it reactive and thereby a suitable chemical feedstock
Treatment of post washing hydrolyzate with sulfur dioxide and heat to maximize the yield of fermentable sugars and to remove, and/or neutralize, fermentation inhibitors;
Evaporation to remove and recover cooking chemicals, remove side products, precipitate reactive native lignin and concentrate lignosulfonates and/or fermentable sugars product;
Lignin separation to remove reactive native lignin and reactive lignosulfonates from fermentable sugars;
Fermentation and distillation to produce and concentrate aliphatic alcohols or organic acids;
Drying the concentrated aliphatic alcohols or organic acids with anhydrous lime and reusing the resulting hydrated lime byproduct for lignin separation; and
Fractionation and/or separation to remove and recover side products.

Another aspect is a process further comprising the step of fractionation and/or separation to remove and recover side products.

Another aspect is a process wherein aliphatic alcohol-soluble lignin is separated by evaporation of said aliphatic alcohol and subsequent removal of reactive native lignin precipitate.

Another aspect is a process wherein reactive lignosulfonates are selectively precipitated using excess lime in the presence of aliphatic alcohol.

Another aspect is a process wherein lignosulfonates filter cake is combusted in a fluidized bed boiler or gasifier, sulfur is released and reacts with excess lime in said filter cake to form gypsum.

Another aspect is a process further comprising the step of fermentation and distillation.

Another aspect is a process wherein anhydrous lime is used to dry product aliphatic alcohol from distillation and resultant hydrated lime byproduct is reused to displace fresh lime in the associated upstream feedstock preparation process.

Another aspect is a process wherein said hydrated lime byproduct is used to precipitate lignosulfonates.

Another aspect is a process wherein aliphatic alcohol is removed from a stream and the resulting stream is concentrated in an integrated alcohol stripper and evaporator system, wherein the evaporated vapor is compressed using vapor compression and provides the thermal energy for both the stripper and the evaporator.

Another aspect is a process wherein evaporated vapor streams are segregated so as to have different concentrations of organic compounds in different streams.

Another aspect is a process wherein evaporator condensate streams are segregated so as to have different concentrations of organic compounds in different streams.

Another aspect is a process wherein the evaporator condensates and distillation column bottoms are used to wash cellulose to minimize effluent discharges.

Further, the present invention describes a process of fractionating lignocellulosic material into lignin, cellulose and hydrolyzed hemicelluloses through a staged treatment of the lignocellulosic material with a solution of aliphatic alcohol, water and 10-20% wt sulfur dioxide, in multiple step process where:
- the cellulose is first fractionated and in an intermediary step can then be diverted to further treatment,
- the hemicellulose and sugar rich stream, which also contains the cooking chemicals and particularly the aliphatic alcohol, is treated to remove and recycle the aliphatic alcohol and to remove water to concentrate the stream so it is suitable for downstream processing
- the hemicelluloses are converted to fermentable sugars, and fermentation inhibitors are removed
- the native reactive lignin is separated from the hemicelluloses
- the reactive lignosulfonates are separated.

Hence in a preferred embodiment lignocellulosic material is treated in a first stage with aliphatic alcohol, water and 10-20% wt sulfur dioxide, the cellulose is then removed, and then both fractions are each further treated with aliphatic alcohol, water, sulfur dioxide or acid. Aliphatic alcohol is stripped and removed from the resulting liquid stream and then the stream is concentrated in an integrated alcohol stripper and evaporator system, wherein the evaporated vapor is compressed using vapor compression and provides the thermal energy for both the stripper and the evaporator. Alcohol soluble lignin is separated from the precipitate or sulfonated by adding sulfur dioxide and heat. Addition of hydrated lime will precipitate alkali-insoluble lignosulfonates. Remaining sugar solution is fermented and distilled of ethanol. Distillation column bottoms and condensate are used for pulp washing. Finally the product ethanol is refluxed over lime to remove water from azeotropic solution.

### BRIEF DESCRIPTION OF THE DRAWINGS

A more complete understanding of the present invention may be obtained by reference to the following detailed description when read in conjunction with the accompanying drawings wherein:

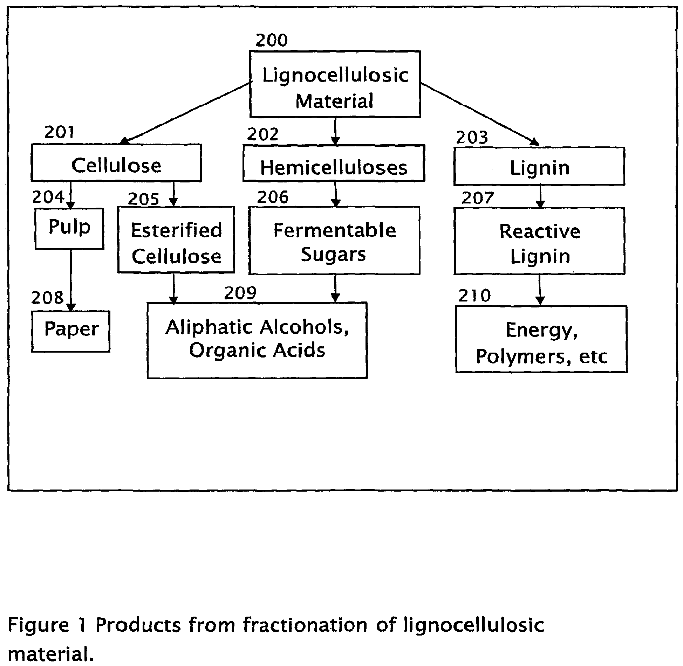

Figure 1. Illustrates the products obtained from the fractionation of lignocellulosic material;
Figure 2. Illustrates a flow sheet example of the process disclosed herein, noting that the process steps may be in other sequences and
Figure 3. Illustrates a flow sheet example of the integrated alcohol stripper and evaporator system disclosed herein.

### DETAILED DESCRIPTION OF THE INVENTION

A method and system is disclosed herein for the fractionation of lignocellulosic materials into reactive chemical feedstock in a batch or semi-continuous process by the stepwise treatment with aqueous aliphatic alcohols in the presence of sulfur dioxide or acid. Lignocellulosic material is fractionated in a fashion that cellulose is removed as pulp. or converted to esterified cellulose, cooking chemicals are reused, lignin is separated in the forms of reactive native lignin and reactive lignosulfonates and hemicelluloses are converted into fermentable sugars, while fermentation inhibitors are removed. In an integrated vapor compression stripper and evaporator system, aliphatic alcohol is removed from a liquid stream and the resulting stream is concentrated for further processing.

A process is provided for fractionating lignocellulosic material into chemically reactive components through a staged treatment of the lignocellulosic material with a solution of aliphatic alcohol, water and 10-20% wt sulfur dioxide with intermediate separation of cellulose and hemicelluloses-lignin fractions, wherein said process further comprises a step of lignin separation and a step wherein the hemicelluloses are converted into fermentable sugars, comprising the steps of:
Cooking under acidic conditions to produce hydrolyzed hemicelluloses, cellulose, reactive lignin and sulfonated lignin, wherein the concentration of sulfur dioxide and aliphatic alcohol in the solution and the time of cook is varied to control the yield of hemicelluloses vs. celluloses and vs. fermentable sugars;
Washing to separate lignin and hemicelluloses from cellulose in several stages to recover over 95% of the aliphatic alcohol mixed with the cellulose;
Diverting the cellulose to papermaking or treatment of cellulose with an aqueous solution of aliphatic alcohol in the presence of acid to esterify the cellulose. rendering it reactive and thereby a suitable chemical feedstock;
Treatment of post washing hydrolyzate with sulfur dioxide and heat to maximize the yield of fermentable sugars and to remove, and/or neutralize fermentation inhibitors;
Evaporation to remove and recover cooking chemicals, remove side products, precipitate reactive native lignin and concentrate lignosulfonates and/or fermentable sugars product;
Lignin separation to remove reactive native lignin and reactive lignosulfonates from fermentable sugars;
Fermentation and distillation to produce and concentrate aliphatic alcohols or organic acids;
Drying the concentrated aliphatic alcohols or organic acids with anhydrous lime and reusing the resulting hydrated lime byproduct for lignin separation; and
Fractionation and/or separation to remove and recover side products.

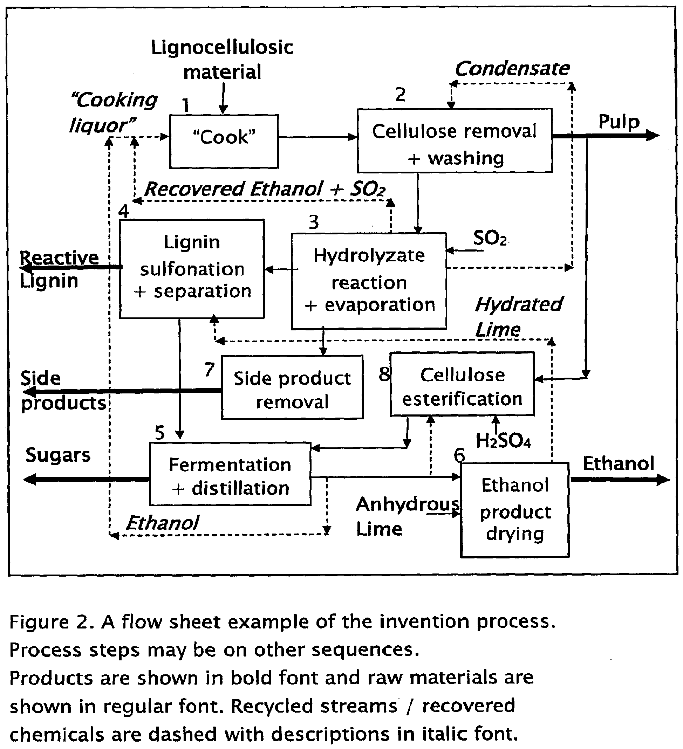

The first process step is "cooking", element 1 in Figure 2, which fractionates the lignocellulosic material components to allow easy downstream removal; specifically hemicelluloses are dissolved and over 50% are completely hydrolyzed, cellulose is separated but remains resistant to hydrolysis, and lignin is sulfonated in water soluble form. Lignocellulosic material is processed, "cooked", in a solution of aliphatic alcohol, water, and sulfur dioxide where typical ratios by weight are 40-60% of both aliphatic alcohol and water, and 10-20% wt of sulfur dioxide; this solution is termed cooking liquor. Aliphatic alcohols can include ethanol, methanol, propanol and butanol, but preferably ethanol. The cooking is performed in one or more stages using batch or continuous digesters. Depending on the lignocellulosic material to be processed, the cooking conditions are varied, with temperatures from 65 °C to 200 °C, for example 65 °C, 75 °C, 85 °C, 95 °C, 105 °C, 115 °C, 125 °C, 130 °C, 135 °C, 140 °C, 145 °C, 150 °C, 155 °C, 165 °C, 170 °C, 180 °C, 190 °C or 200 °C, and corresponding pressures from 1 atmosphere to 15 atmospheres. The sulfur dioxide charge in the cooking liquor is varied between 10% and 20%, for example 10%, 11%, 12%, 13%,

14%, 15%, 16%, 17%, 18%, 19% or 20% of the total cooking liquor mass in one or more cooking stages. Cooking time of each stage is also varied between 15 minutes and 720 minutes, for example 15, 30, 45, 60, 90, 120, 140, 160, 180, 210, 240, 270, 300, 330, 360, 390, 420, 450, 480, 510, 540, 570, 600, 630, 660, 690 or 720 minutes. The lignocellulosic materialto cooking liquorratiocan be varied between 1:3 to 1:6, for example, 1:3, 1:4, 1:5 or 1:6, and preferably 1:4.

Hydrolyzate from the cooking step is subjected to pressure reduction, either at the end of a cook in a batch digester, or in an external flash tank after extraction from a continuous digester. The flash vapor from the pressure reduction is collected into a cooking liquor make-up vessel. The flash vapor contains substantially all the unreacted sulfur dioxide which is directly dissolved into new cooking liquor. The cellulose is then removed to be washed and further treated as required.

The process washing step, element 2 in Figure 2, recovers the hydrolyzate from the cellulose. The washed cellulose is pulp that can be used for paper production or esterification. The weak hydrolyzate from the washing step continues to the post washing hydrolyzate reaction step, element 3 in Figure 2; in a continuous digester application this weak hydrolyzate will be combined with the extracted hydrolyzate from the external flash tank.

In the post washing hydrolyzate reaction step, the post washing hydrolyzate is further treated in one or multiple steps with a solution of aliphatic alcohol, water, and sulfur dioxide, sulfurous acid or sulfuric acid, where typical ratios by weight of aliphatic alcohol to water is between 1:99 and 50:50, for example 1:99, 2:98, 3:97, 4:96, 5:95, 10:90, 20:80, 30:70, 40:60, and 50:50, and sulfur dioxide, sulfurous acid or sulfuric acid to a charge of 0.5% and 20%, for example 0.5%, 1%, 1.5%, 2%, 2.5%, 3%, 3.5%, 4%, 5%, 6%, 7%, 8%, 9%, 10%, 11 %, 12%, 13%, 14%, 15%, 16%, 17%, 18%, 19% or 20%, and directly or indirectly heated to temperatures up to 200 °C, for example 105 °C, 115 °C, 125 °C, 135 °C, 140 °C, 145 °C, 150 °C, 155 °C, 160 °C, 170°C, 180°C, 190°C or 200 °C, and preferably 140 °C. Said solution may or may not contain residual alcohol. This step produces fermentable sugars which can then be concentrated by evaporation to a fermentation feedstock. Concentration by evaporation can be before or after the treatment with sulfur dioxide, sulfurous or sulfuric acid in said post washing hydrolyzate reaction step. This step may or may not be followed by steam stripping of the resultant hydrolyzate to remove and recover sulfur dioxide and aliphatic alcohol and for removal of potential fermentation inhibiting side products. The evaporation process may be under vacuum or pressure from -0.1 atmospheres to 3.0 atmospheres, for example 0.1, 0.3, 0.5, 1.0, 1.5, 2.0,
2.5, or 3.0 atmospheres. Aliphatic alcohol is recovered from the evaporation process by condensing the exhaust vapor and is returned to the cooking liquor make-up vessel in the cooking step. Clean condensate from the evaporation process is used in the washing step. The hydrolyzate from the evaporation and post washing hydrolyzate reaction step contains mainly fermentable sugars but may also contain lignin depending on the location of the lignin separation step in the overall process configuration, and is concentrated between 10% and 55% solids, for example 10%, 15%, 20%, 25%, 30%, 35%, 40%, 45%, 50% or 55%; this hydrolyzate continues to a subsequent process step. In a preferred embodiment the evaporation step comprises the present invention's integrated alcohol stripper and evaporator.

Fermentable sugars are defined as hydrolysis products of cellulose, galactoglucomannan, glucomannan, arabinogalactan, arabinoglucuronoxylans, and glucuronoxylans in to their respective short-chained oligomer and monomer products, i.e., glucose, mannose, galactose, xylose, and arabinose, which are substantially free of fermentation inhibitors. In a preferred embodiment, this is a solution of monomer sugars essentially free of fermentation inhibitors. In a most preferred embodiment it is a solution of monomer sugars with concentration of furfural below 0.15% of the sugars.

Cellulose removed in the washing step can be diverted for papermaking or in a preferred embodiment can be esterified into reactive cellulose by further treatment with aliphatic alcohol in the presence of sulfur dioxide or acid, element 8 in Figure 2. Aliphatic alcohol will be at a concentration of 96% or higher in the presence of sulfur dioxide, sulfurous acid or sulfuric acid to a charge of 0.5% and 20%, for example 0.5%, 1%, 1.5%, 2%, 2.5%, 3%, 3.5%, 4%, 5%, 6%, 7%, 8%, 9%, 10%, 11%, 12%, 13%, 14%, 15%, 16%, 17%, 18%, 19% or 20%, and directly or indirectly heated to temperatures up to 200 °C, for example 100 °C, 105 °C, 110 °C, 115 °C, 120 °C, 125 °C, 130 °C, 140 °C, , 150 °C, 160 °C 170°C, 180 °C, 190 °C or 200 °C, and preferably 120 °C. The reactive cellulose can either be sold for use as reactive chemical feed stock, or be fermented into an aliphatic alcohol or organic acid.

The process lignin separation step, element 4 in Figure 2, is for the separation of lignin from the hydrolyzate and can be located before or after the post washing hydrolyzate reaction step and evaporation. If located after, then reactive native lignin precipitates from the hydrolyzate since aliphatic alcohol has been removed in the evaporation step. The remaining water soluble lignosulfonates are precipitated by converting the hydrolyzate to an alkaline condition using an alkaline earth oxide, preferably fresh lime or, in a preferred embodiment, hydrated lime from the product aliphatic alcohol drying step. The alkaline condition is due to presence of unreacted lime, termed excess lime. The combined lignin and lignosulfonate precipitate is filtered. The lignin and lignosulfonate filter cake can be dried as a saleable byproduct or be burned in a fluidized bed boiler or gasifier for energy production; sulfur released by combustion reacts with excess lime in the filter cake to form gypsum which can be collected and sold as a side product. The hydrolyzate from filtering can either be sold as a concentrated sugar solution product or be further processed in a subsequent fermentation step to aliphatic alcohol.

The process fermentation and distillation step, element 5 in Figure 2, is for the production of aliphatic alcohols, most preferably ethanol, or organic acids. After removal of cooking chemicals and lignin, and treatment in the post washing hydrolyzate reaction step, the hydrolyzate contains mainly fermentable sugars in water solution from which any fermentation inhibitors have been removed or neutralized. The hydrolyzate is fermented to produce dilute alcohol or organic acids, from 1% to 10% concentration. Spent yeast is removed by filtration. The dilute alcohol is distilled to concentrate to near to its azeotropic point of 95.6% by weight. Some of the alcohol produced from this stage is used for the cooking liquor makeup in the process cooking step. The majority of the alcohol produced is excess and is purified for saleable grade product in the product ethanol drying step. In a preferred embodiment the distillate column bottoms solution and evaporator condensates are used to wash cellulose in the process washing step to minimize effluent discharges.

The aliphatic alcohol product drying step, element 6 in Figure 2, is for the removal of the water from aliphatic alcohol-water azeotrope. After distillation of the aliphatic alcohol, the remaining water is removed by anhydrous lime, where product aliphatic alcohol vapor is released through a vertical absorption column containing anhydrous lime. Hydrated lime is withdrawn from the bottom of the column in a batch or continuous manner, as a byproduct and can be reused to displace fresh lime in the associated upstream feedstock preparation process. In a preferred embodiment the hydrated lime will be used for precipitation in the lignin separation step, element 4 in Figure 2. The product aliphatic alcohol is purified to fuel grade alcohol by use of molecular sieves.

The process side products removal step, element 7 in Figure 2, uses fractionation or separation techniques to remove side products from the hydrolyzate that are of economic value or accumulate to inhibit the yield and quality of the alcohol or pulp products. These side products are isolated by processing the vent from the final reaction step and the condensate from the evaporation step. Side products include furfural, methanol,and acetic acid.

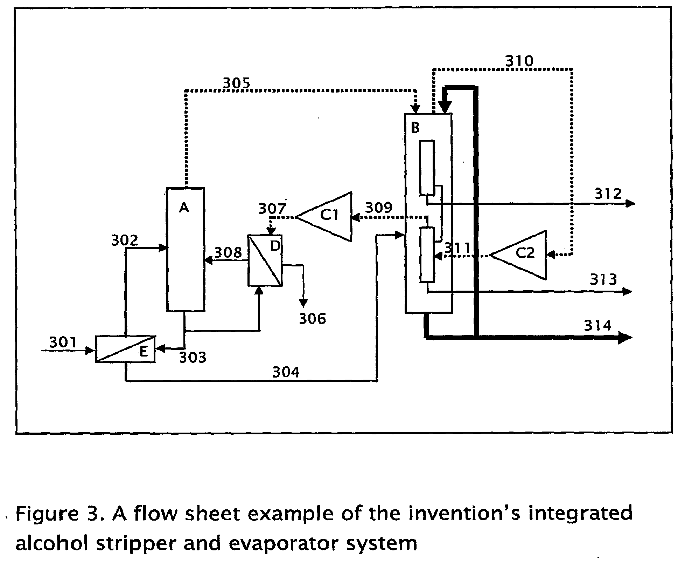

A system is disclosed herein for removing aliphatic alcohol from a stream and concentrating the resulting stream comprising an integrated alcohol stripper and evaporator system, as illustrated in the flow sheet provided in Figure 3.wherei n aliphatic alcohol is removed by vapor stripping, the resulting stripper product stream is concentrated by evaporating water from the stream, evaporated vapor is compressed using vapor compression and is reused to provide thermal energy.for both the stripper and the evaporator.

In Figure 3 the following reference numerals refer to the indicated elements:

| Ref. No. | | Elements |
|---|---|---|
| 301. | | Stripper feed. |
| 302. | | Preheated stripper feed. |
| 303. | | Stripper column bottoms. |
| 304. | | Cooled stripper column bottoms. |
| 305. | | Stripper column overhead. |
| 306. | | Foul condensate. |
| 307. | | Reboiler steam supply. |
| 308. | | Reboiler to stripper column, or direct steam supply. |
| 309. | | Evaporator vapor to stripper column vapor compressor. |
| 310. | | Evaporator vapor to evaporator vapor compressor. |
| 311. | | Evaporator steam supply. |
| 312. | | Aliphatic alcohol. |
| 313. | | Evaporator condensate; up to 4 condensate streams of varying levels of contamination. |
| 314. | | Concentrated organic stream. |
| | A. | Stripper column. |
| | B. | Evaporator. |
| | C1. | Stripper column vapor compressor. |
| | C2. | Evaporator vapor compressor. |
| | D. | Reboiler. |
| | E. | Stripper feed heat exchanger. |

The stripper feed (301) is any stream containing aliphatic alcohol for which it is desired to remove and capture the aliphatic alcohol and to concentrate the remaining organic stream for further processing. This may be the weak hydrolyzate from the current invention's process washing step; in a continuous digester application this weak hydrolyzate will be combined with the extracted hydrolyzate from the external flash tank to form the stripper feed. Alternatively, this may be the feed to the beer column in a corn ethanol plant.

The stripper feed is dilute with a concentration between 0.5 and 50% in non-water components by weight, for example 0.5%, 1%, 2%, 3%, 4%, 5%, 6%, 7%, 8%, 9%, 10%, 11 %, 12%, 13%, 14%, 15%, 16%, 17%, 18%, 19%, 20%, 21%, 22%, 23%, 24%, 25%, 30%, 35%, 40%, 45%, and 50% of which aliphatic alcohol is present in concentration between 0.1 % and 25% for example 0.1%, 0.5%, 1%, 2%, 3%, 4%, 5%, 6%, 7% 8%, 9%, 10%, 11 %, 12%, 13%, 14%, 15%, 16%, 17%, 18%, 19%, 20%, 21%, 22%. 23%, 24%, and 25%.

The stripper feed is first preheated with the stripper column bottoms (303) in the stripper feed heat exchanger (E) and then directed to the stripper column (A). The stripper column (A) can be operated with a reboiler (0) or by direct steam injection. The stripper column overhead (305) is directed to the evaporator (B) which also acts as a reflux condenser where the stripper column overhead is condensed. 'The stripper column bottoms (304), after being cooled by heat exchanging with the stripper feed, is directed to the evaporator (8), where it is evaporated. The resulting evaporated vapor is removed from the evaporator and separated into two vapor streams (309) and (310); one (309) is compressed in the stripper column vapor compressor (C1) and directed to the stripper reboiler to provide the required thermal energy for the stripper column,while the other (310) is compressed in the evaporator vapor compressor (C2) and directed to the evaporator to provide the required steam supply.

From one to four different streams of condensate are segregated so as to have different concentrations of organic compounds in different streams, and are removed from the evaporator (313). One of these streams, usually the cleanest (i.e. the one with the highest water concentration), may be used in the
process washing step, or in another step in the process where a clean condensate stream is required. The other condensate streams are richer in organic compounds, for example, acetic acid, methanol, furfural, aldonic acids and others, and are directed to the fractionation step. The foul condensate (306) from the reboiler can also be directed to the process fractionation step.

The operating conditions of the stripper column are such that the rejected heat from the evaporator is of high enough temperature to drive evaporation. The innovation lies in the integration and order of the operations where first the aliphatic alcohol is removed and second the resulting stream is concentrated, while both operations are driven by steam produced from compressing the evaporated vapor to two different operating pressures. As such the stripper must be operated at higher pressure than the evaporator and specifically at pressures between 0.34 and 12 atmospheres for example at 0.34, 0.40, 0.48, 0.54, 0.61, 0.68, 0.75, 0.82, 0.88, 0.95, 1.02, 1.09, 1.16, 1.22, 1.29, 1.36, 1.43, 1.50, 1.57, 1.63, 1.70, 1.77, 1.84, 1.91, 1.97, 2.04, 2.11, 2.18, 2.25, 2.31, 2.38, 2.45, 2.52, 2.59, 2.65, 2.72, 2.79, 2.86, 2.93, 2.99, 3.06, 3.13, 3.20, 3.27, 3.33, 3.40, 3.47, 3.54, 3.61, 3.67, 3.74, 3.81, 3.88, 3.95, 4.01, 4.08, 4.15, 4.22, 4.29, 4.35, 4.42, 4.49, 4.56, 4.63, 4.70, 4.76, 5.10, 5.44, 5.78, 6.12, 6.46, 6.80, 7.14, 7.49, 7.83, 8.17, 8.51, 8.85, 9.19, 9.53, 9.87, 10.21, 10.55, 10.89, 11.23, 11.57, 11.91 atmospheres while the evaporator operates between -1.00 to 16.80 atmospheres, for example -1.00, -0.95, - 0.92, -0.88, -0.85, -0.82, -0.78, -0.75, -0.71, -0.68, -0.65, -0.61,-0.58, -0.54, -0.51, -0.48, - 0.44, -0.41, -0.37, -0.34, -0.31,-0.27, -0.24, -0.20, -0.17, -0.14, -0.10, -0.07, -0.03, 0, 0.03, 0.07, 0.10, 0.14, 0.17, 0.20, 0.24, 0.27, 0.31,0.34, 0.37, 0.41, 0.44. 0.48, 0.51,0.54, 0.58, 0.61, 0.65, 0.68, 0.71,0.75, 0.78, 0.82, 0.85, 0.88, 0.92, 0.95, 0.99, 1.02, 1.05, 1.09, 1.12, 1.16, 1.19, 1.22, 1.26, 1.29, 1.33, 1.36, 1.39, 1.43, 1.46, 1.50, 1.57, 1.63, 1.70, 1.77, 1.84, 1.91, 1.97, 2.04,2.11, 2.18, 2.25, 2.31, 2.38, 2.45, 2.52, 2.59, 2.65, 2.72, 2.79, 2.86, 2.93, 2.99, 3.06, 3.13, 3.20, 3.27, 3.33, 3.40, 3.47, 3.54, 3.61, 3.67, 3.74,
3.81, 3.88, 3.95, 4.01, 4.08, 4.15, 4.22, 4.29, 4.35, 4.42, 4.49, 4.56, 4.63, 4.70, 4.76, 5.10, 5.44, 5.78, 6.12, 6.46, and 6.80 atmospheres.

Electrical power is used to drive the vapor compressors (C1) and (C2) to compress the evaporator vapor to the conditions needed to operate the stripper column and the evaporator. Depending on the temperature of the stripper feed and the operating pressures of the stripper and the evaporator the operations may have a shortfall or an excess of heat. In the former case a live steam supply raised in a boiler outside the process described herein is required to supplement the compressed
evaporator vapor streams. A reboiler may be included to raise low pressure clean steam, i.e., less than 4.42 atmospheres, from condensing the stripper column overhead fraction. The clean steam is then compressed to run the evaporator. In this case the reboiler condensate will be combined with any of the evaporator condensates to the process fractionation step or be directed to the process fractionation step on its own.

The evaporator tubes (the space in the evaporator where the vapor is released) may be separated into two or more spaces, so that the evaporated vapor streams can be segregated and the concentration of the organic compounds in each released vapor stream can be varied. In this case a selection of the more contaminated vapor may be preferably directed to the stripper reboiler.

### Example 1

One representative example of a design heat and material balance of the alcohol stripper and evaporator system is given in the following table:

| Refer To Fig. 3 | | | | | | | Condensate | | | | | | Regenerated Alcohol | Condensate | Sugar Product |
|---|---|---|---|---|---|---|---|---|---|---|---|---|---|---|---|
| Stream | No. | 301 | 302 | 303 | 304 | 305 | 306 | 307 | 308 | 309 | 310 | 311 | 312 | 313 | 314 |
| H₂O | kg/h | 392 | 389 | 380 | 380 | 12 | 125 | 125 | | 125 | 81 | 81 | 12 | 70 | 176 |
| EtO | kg/h | 50 | 50 | 3 | 3 | 48 | 0.4 | 0.4 | | 0.4 | 0.3 | 0.3 | 48 | 1.9 | 0.2 |
| Organics | kg/h | 44 | 47 | 44 | 44 | 0 | 0.05 | 0.05 | | 0.05 | 0.2 | 0.2 | -0.5 | 0.4 | 44 |
| Total | kg/h | 486 | 486 | 427 | 427 | 59 | 126 | 126 | | 126 | 82 | 82 | 59 | 81 | 220 |
| Temp. | °C | 97 | 119 | 144 | 106 | 48 | 146 | 177 | | 101 | 101 | 121 | 119 | 101 | 100 |
| Sat. Temp | °C | | | | | 106 | | 146 | | | | 121 | | | |
| Pressure | atm | | | | | | | 4.42 | | 0 | 0 | 0.34 | | | |
| Et conc. | % | 10.3 | 10.3 | 0.6 | 0.6 | 80.2 | 0.3 | 0.3 | | 0.3 | 0.3 | 0.3 | 80.8 | 2.3 | 0.1 |
| "Solids" | % | 19.3 | 20.0 | 10.9 | 10.9 | 80.2 | 0.4 | 0.4 | | 0.4 | 0.6 | 0.6 | 80.0 | 2.8 | 20.2 |
| "Sugars" | % | 9.0 | 9.7 | 10.4 | 10.4 | 0.0 | 0.0 | 0.0 | | 0.0 | 0.2 | 0.2 | -0.8 | 0.4 | 20.1 |

### Example 2

The following example illustrates a process for producing ethanol from hemicelluloses: - Wood chips of mixed northern pine species, containing 42.68% moisture, were cooked for 180 minutes at 157°C in a 1 liter Parr reactor. The moisture adjusted cooking liquor consisted of 3% SO2, 48.5% of ethanol and 48.5% water by weight in 6 parts of total liquor to 1 part of dry wood.
Cellulose was removed representing 37.1%of the original wood mass.

The monomer sugars represented 61% of the all sugars in the hydrolyzate as determined by autoclaving the hydrolyzate with 4% H₂SO₄ in 121 °C for 60 minutes, which converted the remaining sugars in their corresponding monomers.

Half of the hydrolyzate was processed without the final reaction step. Calcium oxide was added to reach pH of 11 in the hydrolyzate and the precipitate containing calcium lignosulfonates was filtered off. The cooking ethanol was distilled off until the boiling point of the distillate reached 100.5 °C and density of 0.995 g/mL. The furfural content was determined to be 0.29 g/L in the untreated hydrolyzate after the lignin removal and evaporation step.

The second half of the hydrolyzate was subjected to the final reaction step by injecting 3 % by weight of sulfur dioxide and heating for 30 minutes at 140 °C. Calcium oxide was added to reach pH of 11 in the hydrolyzate and the precipitate containing calcium lignosulfonates was filtered off. The cooking ethanol was distilled off until the boiling point of the distillate reached 100.5 °C and density of 0.995 g/mL. The furfural content was determined to be 0.06 g/L in the hydrolyzate after the final processing step.

The untreated hydrolyzate, i.e., that was not subjected to the final reaction step, and the treated hydrolyzate, i.e., that was subjected to the final reaction step, were both prepared for fermentation by neutralizing with acetic acid, adding sodium citrate and commercial nutrient broth. Initial sugar composition and subsequent hydrolyzate composition were determined in HPLC.

Fermentation of both hydrolyzates was performed in a laboratory setting using saccharomyces cerevisiae yeast for at least 72 hours at 35 °C.

The yield of ethanol from the untreated hydrolyzate corresponded to only 18.6% stoichiometric yield of the original oligomer sugars and monomer sugars present in the hydrolyzate.

**Table 1. Monomer sugar concentration of the hydrolyzate and the product ethanol concentration as a function of fermentation time for the untreated hydrolyzate.**

| | **Glucose** | **Xylose** | **Galactose** | **Arabinose** | **Mannose** | **Total Sugars** | **Ethanol** |
|---|---|---|---|---|---|---|---|
| **Fermentation Time (hours)** | **Cone. (g/L)** | **Conc. (g/L)** | **Cone. (g/L)** | **Cone. (g/L)** | **Cone. (g/L)** | **Cone. (g/L)** | **Conc. (g/L)** |
| 0 | 9.33 | 11.83 | 5.30 | 1.94 | 12.05 | 40.45 | 0.00 |
| 24 | 7.55 | 13.91 | 6.17 | 1.69 | 13.22 | 42,54 | 3.76 |
| 48 | 5.85 | 14.79 | 6.71 | 1.84 | 13.48 | 42.67 | 5.57 |
| 72 | 4.41 | 14.74 | 6.68 | 1.74 | 12.72 | 40.29 | 6.30 |

The yield of ethanol from the hydrolyzate treated in the final processing step corresponded to 46.5% stoichiometric yield of the original monomer and oligomer sugars in the hydrolyzate or 2.5 times greater than the amount from the untreated hydrolyzate.

**Table 2. Monomer sugar concentration of the hydrolyzate and the product ethanol concentration as a function of fermentation time for the hydrolyzate treated in the final reaction step.**

| | Glucose | Xylose | Galactose | Arabinose | Mannose | Total Sugars | Ethanol |
|---|---|---|---|---|---|---|---|
| Fermentation Time (hours) | Conc. (g/L) | Conc. (g/L) | Conc. (g/L) | Conc. (g/L) | Conc. (g/L) | Conc. (g/L) | Conc. (g/L) |
| 0 | 8.85 | 10.34 | 4.63 | 1.77 | 10.99 | 36.58 | 0.00 |
| 24 | 4.31 | 9.23 | 4.13 | 1.19 | 8.81 | 27.67 | 3.53 |
| 48 | 0.99 | 9.79 | 4.47 | 1.22 | 7.24 | 23.71 | 7.05 |
| 72 | 0.00 | 6.76 | 3.22 | 1.89 | 3.05 | 14.22 | 14.21 |

## Claims

1. A process for fractionating lignocellulosic material in to chemically reactive components comprising: a staged treatment of the lignocellulosic material with a solution of aliphatic alcohol, water and 10-20% wt sulfur dioxide with intermediate separation of cellulose and hemicelluloses-lignin fractions which are then each further treated, wherein said process further comprises a step of lignin separation and a step wherein the hemicelluloses are converted into fermentable sugars.

2. A process according to claim 1 wherein said solution of aliphatic alcohol, water and sulfur dioxide contains 40% to 60% water.

3. A process according to claim 1 or 2, wherein a different concentration of said solution of aliphatic alcohol, water and sulfur dioxide is used at a first stage of treatment of said lignocellulosic material than is used in one or more subsequent stages of treatment with intermediate removal and preservation of cellulose.

4. A process according to any one of claims 1 to 3, wherein a sulfur dioxide, sulfurous acid or sulfuric acid solution of 0.5% to 20% is used in one or more subsequent stages of treatment with intermediate removal and preservation of cellulose.

5. A process according to any one of claims 1 to 4, wherein said process is followed by steam stripping and/or evaporation of hydrolyzate to remove and recover sulfur dioxide and aliphatic alcohol and to remove fermentation inhibitors.

6. A process according to any one of claims 1 to 5, wherein cellulose is further treated with an aqueous solution of aliphatic alcohol in the presence of acid to esterify and render it a reactive chemical feedstock.

7. A process according to claim 6, wherein esterified cellulose is fermented to aliphatic alcohol.

8. A process according to any one of claims 1 to 7, wherein said process is carried out at temperatures between 65°C and 200°C.

9. A process according to any one of claims 1 to 8, wherein said process is carried out at for a period of time between 15 minutes and 720 minutes.

10. A process according to any one of claims 1 to 9, wherein said process results in the production of fermentable sugars from the hemicelluloses of said lignocellulosic material.

11. A process according to any one of claims 1 to 10 wherein aliphatic alcohol is produced from fermenting and distilling hydrolyzed fermentable sugars produced in said process and is then reused in said process.

12. A process according to any one of claims 1 to 11, wherein lignin is sulfonated and rendered soluble in aqueous solutions.

13. A process according to any one of claims 1 to 12, wherein the concentration of sulfur dioxide and aliphatic alcohol in the solution and the time of cook is varied to control the yield of hemicelluloses vs. celluloses and vs. fermentable sugars.

14. A process according to any one of claims 1 to 13, wherein excess sulfur dioxide is released from said further treatment of each fraction and used for make-up for cooking chemicals.

15. A process according to any one of claims 1 to 14 comprising the steps of:
a) Cooking under acidic conditions to produce hydrolyzed hemicelluloses, cellulose, reactive lignin and sulfonated lignin;
b) Washing to separate lignin and hemicelluloses from cellulose in several stages to recover over 95% of the aliphatic alcohol mixed with the cellulose;
c) Diverting the cellulose to papermaking or treatment of cellulose with an aqueous solution of aliphatic alcohol in the presence of acid to esterify the cellulose, rendering it reactive and thereby a suitable chemical feedstock;
d) Treatment of post washing hydrolyzate with sulfur dioxide and heat to maximize the yield of fermentable sugars and to remove, and/or neutralize fermentation inhibitors;
e) Evaporation to remove and recover cooking chemicals, remove side products, precipitate reactive native lignin and concentrate lignosulfonates and/or fermentable sugars product;
f) Lignin separation to remove reactive native lignin and reactive lignosulfonates from fermentable sugars;
g) Fermentation and distillation to produce and concentrate aliphatic alcohols or organic acids;
h) Drying the concentrated aliphatic alcohols or organic acids with anhydrous lime and reusing the resulting hydrated lime byproduct for lignin separation; and
i) Fractionation and/or separation to remove and recover side products.

16. A process according to any one of claims 1 to 15, further comprising the step of fractionation and/or separation to remove and recover side products, the step of lignin and/or lignosulfonate separation, the separation of aliphatic alcohol soluble lignin by evaporation of said aliphatic alcohol and subsequent removal of reactive native lignin precipitate, the selective precipitation of reactive lignosulfonates using excess lime in the presence of aliphatic alcohol, the combustion of lignosulphonates filter cake in a fluidized bed boiler or gasifier, and reaction of released sulfur with excess lime in said filter cake to form gypsum, and/or the step of fermentation and distillation.

17. A process according to any one of claims 1 to 16, wherein lignosulfonates are precipitated using hydrated lime produced from the reaction of anhydrous lime and product aliphatic alcohol.

18. A process according to any one of claims 1 to 17, wherein evaporated vapour streams are segregated so as to have different concentrations of organic compounds in different streams, or wherein evaporator condensate streams are segregated so as to have different concentrations of organic compounds in different streams.

## Patentansprüche

1. Verfahren zum Fraktionieren von Lignozellulosematerial in chemisch reaktive Komponenten, umfassend: eine Stufen-Behandlung des Lignozellulosematerials mit einer Lösung von aliphatischem Alkohol, Wasser und 10-20 Gew.-% Schwefeldioxid mit Zwischenabtrennung von Zellulose- und Hemizellulose-Lignin-Fraktionen, die dann jeweils weiter behandelt werden, wobei das Verfahren weiter einen Schritt einer Lignin-Abtrennung und einen Schritt, bei dem die Hemizellulosen in fermentierbare Zucker umgewandelt werden, umfasst.

2. Verfahren nach Anspruch 1, wobei die Lösung von aliphatischem Alkohol, Wasser und Schwefeldioxid 40% bis 60% Wasser enthält.

3. Verfahren nach Anspruch 1 oder 2, wobei eine verschiedene Konzentration der Lösung von aliphatischem Alkohol, Wasser und Schwefeldioxid bei einer ersten Stufe der Behandlung des Lignozellulosematerials verwendet wird, als in einer oder mehreren anschließenden Stufen der Behandlung mit Zwischenentfernung und Konservierung von Zellulose verwendet wird.

4. Verfahren nach einem der Ansprüche 1 bis 3, wobei eine Schwefeldioxid-, schweflige Säure- oder Schwefelsäure-Lösung von 0,5% bis 20% in einer oder mehreren anschließenden Stufen der Behandlung mit Zwischenentfernung und Konservierung von Zellulose verwendet wird.

5. Verfahren nach einem der Ansprüche 1 bis 4, wobei dem Verfahren Dampfabstreifen und/oder Verdampfung von Hydrolysat zum Entfernen und Gewinnen von Schwefeldioxid und aliphatischem Alkohol und zum Entfernen von Fermentationsinhibitoren folgt.

6. Verfahren nach einem der Ansprüche 1 bis 5, wobei Zellulose mit einer wässrigen Lösung von aliphatischem Alkohol in Gegenwart von Säure weiter behandelt wird, um sie zu verestern und ein reaktives chemisches Ausgangsmaterial zu erzeugen.

7. Verfahren nach Anspruch 6, wobei veresterte Zellulose zu aliphatischem Alkohol fermentiert wird.

8. Verfahren nach einem der Ansprüche 1 bis 7, wobei das Verfahren bei Temperaturen zwischen 65°C und 200°C ausgeführt wird.

9. Verfahren nach einem der Ansprüche 1 bis 8, wobei das Verfahren für einen Zeitraum zwischen 15 Minuten und 720 Minuten ausgeführt wird.

10. Verfahren nach einem der Ansprüche 1 bis 9, wobei das Verfahren die Herstellung von fermentierbaren Zuckern aus den Hemizellulosen des Lignozellulosematerials ergibt.

11. Verfahren nach einem der Ansprüche 1 bis 10, wobei aliphatischer Alkohol durch Fermentieren und Destillieren aus in dem Verfahren hergestelltem hydrolysierten fermentierbaren Zuckern hergestellt wird und dann in dem Verfahren wiederverwendet wird.

12. Verfahren nach einem der Ansprüche 1 bis 11, wobei Lignin sulfoniert und in wässrigen Lösungen löslich gemacht wird.

13. Verfahren nach einem der Ansprüche 1 bis 12, wobei die Konzentration von Schwefeldioxid und aliphatischem Alkohol in der Lösung und die Zeit zum Kochen zum Steuern der Ausbeute von Hemizellulosen gegenüber Zellulosen und gegenüber fermentierbaren Zuckern variiert wird.

14. Verfahren nach einem der Ansprüche 1 bis 13, wobei überschüssiges Schwefeldioxid aus der weiteren Behandlung jeder Fraktion freigesetzt und zum Auffüllen von Kochchemikalien verwendet wird.

15. Verfahren nach einem der Ansprüche 1 bis 14, umfassend die Schritte:
a) Kochen unter sauren Bedingungen zur Herstellung von hydrolysierten Hemizellulosen, Zellulose, reaktivem Lignin und sulfoniertem Lignin;
b) Waschen zum Trennen von Lignin und Hemizellulosen von Zellulose in verschiedenen Stufen zum Gewinnen von über 95% des aliphatischen Alkohols vermischt mit der Zellulose;
c) Abzweigen der Zellulose zur Papierherstellung oder Behandlung von Zellulose mit einer wässrigen Lösung von aliphatischem Alkohol in Gegenwart von Säure zum Verestern der Zellulose, um sie reaktiv und dadurch zu einem geeigneten chemischen Ausgangsmaterial zu machen;
d) Behandlung des Hydrolysats nach dem Waschen mit Schwefeldioxid und Erhitzen zum Maximieren der Ausbeute von fermentierbaren Zuckern und zum Entfernen und/oder Neutralisieren von Fermentationsinhibitoren;
e) Verdampfung zum Entfernen und Gewinnen von Kochchemikalien, Entfernen von Nebenprodukten, Ausfällen von reaktivem nativem Lignin und Konzentrieren von Lignosulfonaten und/oder fermentierbarem Zuckerprodukt;
f) Lignin-Abtrennung zum Entfernen von reaktivem nativem Lignin und reaktiven Lignosulfonaten von fermentierbaren Zuckern;
g) Fermentation und Destillation zum Herstellen und Konzentrieren aliphatischer Alkohole oder organischer Säuren;
h) Trocknen der konzentrierten aliphatischen Alkohole oder organischen Säuren mit wasserfreiem Kalk und Wiederverwenden des erhaltenen hydratisierten Kalknebenprodukts zur Lignin-Abtrennung; und
i) Fraktionierung und/oder Abtrennung zum Entfernen und Gewinnen von Nebenprodukten.

16. Verfahren nach einem der Ansprüche 1 bis 15, weiter umfassend den Schritt der Fraktionierung und/oder Abtrennung zum Entfernen und Gewinnen von Nebenprodukten, den Schritt der Lignin- und/oder Lignosulfonat-Abtrennung, die Abtrennung von in aliphatischem Alkohol löslichem Lignin durch Verdampfung des aliphatischen Alkohols und anschließende Entfernung einer reaktiven nativen Lignin-Ausfällung, wobei die selektive Ausfällung von reaktiven Lignosulfonaten überschüssigen Kalk in Gegenwart von aliphatischem Alkohol anwendet, die Verbrennung von Lignosulfonat-Filterkuchen in einem Wirbelschichtkocher oder Vergaser und Umsetzung von freigesetztem Schwefel mit überschüssigem Kalk in dem Filterkuchen zum Bilden von Gips und/oder den Schritt der Fermentation und Destillation.

17. Verfahren nach einem der Ansprüche 1 bis 16, wobei Lignosulfonate unter Verwendung von hydratisiertem Kalk, hergestellt aus der Reaktion von wasserfreiem Kalk und Produkt von aliphatischem Alkohol, ausgefällt werden.

18. Verfahren nach einem der Ansprüche 1 bis 17, wobei verdampfte Dampfströme segregiert werden, so dass sie verschiedene Konzentrationen organischer Verbindungen in verschiedenen Strömen aufweisen, oder wobei Verdampferkondensatströme segregiert werden, so dass sie verschiedene Konzentrationen organischer Verbindungen in verschiedenen Strömen aufweisen.

## Revendications

1. Procédé de fractionnement de matière lignocellulosique en des composants chimiquement réactifs comprenant : un traitement par étapes du matériau lignocellulosique avec une solution d'alcool aliphatique, d'eau et de dioxyde de soufre à 10-20% en poids avec une séparation intermédiaire de fractions de cellulose et d'hémicellulose-lignine qui sont ensuite traitées davantage chacune, ledit procédé comprenant en outre une étape de séparation de lignine et une étape dans laquelle les hémicelluloses sont converties en sucres fermentables.

2. Procédé selon la revendication 1, dans lequel ladite solution d'alcool aliphatique, d'eau et de dioxyde de soufre contient 40 % à 60 % d'eau.

3. Procédé selon la revendication 1 ou 2, dans lequel une concentration différente de ladite solution d'alcool aliphatique, d'eau et de dioxyde de soufre est utilisée à une première étape de traitement de ladite matière lignocellulosique de celle utilisée dans une ou plusieurs étapes ultérieures de traitement avec une élimination et une préservation intermédiaires de la cellulose.

4. Procédé selon l'une quelconque des revendications 1 à 3, dans lequel une solution de dioxyde de soufre, d'acide sulfureux ou d'acide sulfurique de 0,5 % à 20 % est utilisée dans une ou plusieurs étapes ultérieures de traitement avec une élimination et une préservation intermédiaires de la cellulose.

5. Procédé selon l'une quelconque des revendications 1 à 4, dans lequel ledit procédé est suivi d'une extraction à la vapeur et/ou d'une évaporation d'hydrolysat afin d'éliminer et de récupérer le dioxyde de soufre et l'alcool aliphatique et d'éliminer des inhibiteurs de fermentation.

6. Procédé selon l'une quelconque des revendications 1 à 5, dans lequel la cellulose est ensuite traitée avec une solution aqueuse d'alcool aliphatique en présence d'acide pour l'estérifier et en faire une matière première chimique réactive.

7. Procédé selon la revendication 6, dans lequel la cellulose estérifiée est fermentée en alcool aliphatique.

8. Procédé selon l'une quelconque des revendications 1 à 7, dans lequel ledit procédé est réalisé à des températures comprises entre 65 °C et 200 °C.

9. Procédé selon l'une quelconque des revendications 1 à 8, dans lequel ledit procédé est mis en oeuvre pendant une période de temps entre 15 minutes et 720 minutes.

10. Procédé selon l'une quelconque des revendications 1 à 9, dans lequel ledit procédé conduit à la production de sucres fermentables à partir des hémicelluloses dudit matériau lignocellulosique.

11. Procédé selon l'une quelconque des revendications 1 à 10, dans lequel de l'alcool aliphatique est produit à partir d'une fermentation et d'une distillation de sucres fermentables hydrolysés produits dans ledit procédé et est ensuite réutilisé dans ledit procédé.

12. Procédé selon l'une quelconque des revendications 1 à 11, dans lequel de la lignine est sulfonée et rendue soluble dans des solutions aqueuses.

13. Procédé selon l'une quelconque des revendications 1 à 12, dans lequel la concentration de dioxyde de soufre et d'alcool aliphatique dans la solution et le temps de cuisson sont variés afin de contrôler le rendement en hémicelluloses par rapport aux celluloses et par rapport aux sucres fermentables.

14. Procédé selon l'une quelconque des revendications 1 à 13, dans lequel du dioxyde de soufre excédentaire est libéré dudit traitement ultérieur de chaque fraction et utilisé pour l'appoint des produits chimiques de cuisson.

15. Procédé selon l'une quelconque des revendications 1 à 14, comprenant les étapes de :
a) cuisson dans des conditions acides afin de produire des hémicelluloses hydrolysées, de la cellulose, de la lignine réactive et de la lignine sulfonée ;
b) lavage pour séparer la lignine et les hémicelluloses de la cellulose en plusieurs étapes afin de récupérer plus de 95 % de l'alcool aliphatique mélangé à la cellulose ;
c) transfert de la cellulose à la fabrication de papier ou à un traitement de cellulose avec une solution aqueuse d'alcool aliphatique en présence d'acide pour estérifier la cellulose, la rendant réactive et formant ainsi une matière première chimique appropriée ;
d) traitement de l'hydrolysat de post-lavage avec du dioxyde de soufre et de la chaleur pour maximiser le rendement de sucres fermentables et pour éliminer et/ou neutraliser des inhibiteurs de fermentation ;
e) évaporation afin d'éliminer et de récupérer des produits chimiques de cuisson, éliminer des produits secondaires, précipiter de la lignine native réactive et concentrer un produit de lignosulfonates et/ou de sucres fermentables ;
f) séparation de la lignine afin d'éliminer la lignine native réactive et les lignosulfonates réactifs des sucres fermentables ;
g) fermentation et distillation pour produire et concentrer des alcools aliphatiques ou des acides organiques ;
h) séchage des alcools aliphatiques concentrés ou des acides organiques avec de la chaux anhydre et réutilisation du sous-produit de chaux hydratée résultant pour la séparation de lignine ; et
i) fractionnement et/ou séparation afin d'éliminer et de récupérer des produits secondaires.

16. Procédé selon l'une quelconque des revendications 1 à 15, comprenant en outre l'étape de fractionnement et/ou de séparation afin d'éliminer et de récupérer des produits secondaires, l'étape de séparation de lignine et/ou de lignosulfonates, la séparation de lignine soluble dans de l'alcool aliphatique par évaporation dudit alcool aliphatique et l'élimination subséquente du précipité de lignine native réactive, la précipitation sélective de lignosulfonates réactifs en utilisant de l'excès de chaux en présence d'alcool aliphatique, la combustion d'un gâteau de filtration de lignosulfonates dans une chaudière à lit fluidisé ou un gazéifieur, et la réaction de soufre libéré avec un excès de chaux dans ledit gâteau de filtration pour former du gypse, et/ou l'étape de fermentation et de distillation.

17. Procédé selon l'une quelconque des revendications 1 à 16, dans lequel des lignosulfonates sont précipités en utilisant de la chaux hydratée produite à partir de la réaction de chaux anhydre et d'alcool aliphatique produit.

18. Procédé selon l'une quelconque des revendications 1 à 17, dans lequel des flux de vapeur évaporée sont séparés de manière à avoir différentes concentrations de composés organiques dans des flux différents, ou dans lequel des flux de condensat d'évaporateur sont séparés de manière à avoir différentes concentrations de composés organiques dans différents flux.
